# EUROPEAN PATENT SPECIFICATION

(11) **EP 1 390 397 B1**
(45) Date of publication and mention of the grant of the patent: **01.10.2008**
(21) Application number: 02771774.3
(22) Date of filing: 02.05.2002
(51) Int. Cl.: C07K 14/02, C12N 1/19, C12N 15/81, A61K 39/29

(54) **PRE-S PROTEIN OF HEPATITIS B VIRUS (HBV) AS AN ADJUVANT AND A COMPONENT OF HBV VACCINE**
PRE-S-PROTEIN VON HEPATITIS-B-VIRUS (HBV) ALS ADJUVANS UND KOMPONENTE EINES HBV-IMPFSTOFFS
PROTEINE PRE-S DU VIRUS D'HEPATITE B (HBV) COMME ADJUVANT ET COMPOSANTE DU VACCIN HBV

(30) Priority: 25.05.2001 KR 2001029002
(43) Date of publication of application: 25.02.2004
(73) Proprietor: Dobeel Corp., Kyungki-do (KR)
(72) Inventor: MOON, Hong-Mo, 464-120 Kwangju-si, Kyungki-do (KR); KIM, Ki-Yong, Jungwon-gu, Seongnam-si, Kyungki-do (KR); YUM, Jung-Sun, Jungwon-gu, Seongnam-si, Kyungki-do (KR); AHN, Byung-Cheol, Seongnam-city, Kyungki-do 463-500 (KR); YI, Su-Young, Jungwon-gu, Seongnam-si, Kyungki-do (KR); SEOMUN, Young, Jungwon-gu, Seongnam-si, Kyungki-do (KR); PARK, Ji-Hee, Seoul 137-130 (KR)
(74) Representative: Ritthaler, Wolfgang
(86) International application number: PCT/KR2002/000820
(87) International publication number: WO 2002/094866

(56) References cited:
- EP-A2- 0 154 902
- EP-A2- 0 248 167
- EP-B1- 0 244 924

## Description

### BACKGROUND OF THE INVENTION

### (a) Field of the Invention

The present invention relates to an adjuvant activity of pre-S, and to the use of pre-S as an HBV vaccine component. More specifically, it relates to the function of pre-S in enhancing the immunogenicity of an HBV S antigen and to its use as a component of an improved prophylactic HBV vaccine and a therapeutic vaccine for the treatment of chronic HBV carriers.

### (b) Description of the Related Art

The hepatitis B virus (hereinafter referred to as "HBV") is transmitted primarily through activities that involve contact with blood or blood -derived fluids, and then spreads to other liver cells through the blood.

The majority of individuals who have HBV infection fully recover from the virus infection by the body's immune responses, but about 5% of them become chronic carriers. This becomes worse (over 90%) in the case when infants are infected through a chronic carrier mother or from other sources during the perinatal period. In addition, infection through organ transplantation leads to an almost 100% carrier state. Most patients maintain a carrier condition that shows no severe symptoms, but 10 to 30% of these patients experience chronic hepatitis, they slowly progress to liver cirrhosis, and then eventually develop a hepatocarcinoma.

Once infection of the HBV takes place, it normally progresses to acute hepatitis and strong polyclonal immune responses are produced, which cure the hepatitis naturally. The natural cure is associated with multispecific cytotoxic T lymphocyte ("CTL") responses and a strong polyclonal humoral immune response. That is, efficient neutralization by the virus-specific antibodies and specific killing of the virus-infected cells by induced CTL leads to successful recovery from the infection. In contrast, people who become chronic carriers show weak oligoclonal immune responses. A similar finding was also observed from an experiment with HBV transgenic mice.

Forty-two nm dane particles present in the sera of chronic carriers are the infectious form of the HBV. The other 22 nm particles and rode shaped forms are not infectious. Each infectious particle is composed of an envelope, a capsid (HBcAg), a virus specific DNA polymerase, and a 32 Kb circular double stranded DNA. One other virus specific protein, HBV e antigen (HBeAg), is found in soluble form in the serum.

Three different surface antigens are made from the envelope gene. They are the large hepatitis B surface antigen (L-HBsAg), the middle (M-HBsAg), and the small antigen (S-HBsAg), which is also known as the S-antigen. The large antigen is made from the complete envelope gene encompassing DNA sequence of the pre-S1-, pre-S2- and S-regions, whereas the middle antigen is derived from only the pre-S2- and S-regions while the small antigen is derived from only the S-region. When the composition of these three antigens in the virus particles are quantitatively compared, the S-HBsAg makes up the largest portion, while the M-HBsAg and the L-HBsAg only make up small portions, comprising only 2 to 5% of the total envelope antigens.

All commercially available vaccines against HBV, except one or two, contain the HBV small surface antigen (HBsAg). The authentic HBV S antigen can be recovered from plasma of chronic HBV carrier as particles of about 22 nm composed of two proteins known as P24 and its glycosylated derivative GP28, both of which are encoded by the 226 ami no acid coding sequence on the HBV genome known as the S protein coding sequence, or HBV S-gene. However, both animal and human experiments have shown that the pre -S portion has stronger immunogenicity than the S antigen, forming antibodies much faster than the S antigens (at least 20 weeks faster than the S antigens). In fact, human clinical studies have shown that only two injections with pre-S-containing vaccine could achieve the same level of protection as three injections with S antigen only vaccine. Furthermore, the pre-S-containing vaccine induced antibody responses more effectively for the non-responders compare to the S antigen only vaccines. It is also known that the pre-S protein attaches to the liver cell first during virus infection, probably acting as the virus receptor ligand. If this is true, antibodies against this portion can prevent the infection of new liver cells as well as neutralize the virus.

Despite all these possibilities, mass-production of the pre-S protein has not been established, restricting its use as a vaccine. When the whole envelope gene is expressed in animal cells (e.g. CHO cells), S antigens are mostly expressed so that the pre-S antigen content is as low as 2 to 3%. Producing antigens from animal cells is not cost-effective. Virus particles purified from the sera of chronic carriers of the hepatitis B virus contain pre-S antigens at less than 5% of the total antigen, so this is also not an effective way to produce vaccine antigens, for several obvious reasons.

Currently, interferon and lamivudine are used as treatments for chronic HBV carriers. The efficacy of interferon for the treatment of HBV chronic carriers ranges from 20 to 25%, and approximately 10% of those treated suffer side effects so severe that the treatment must be stopped. Interferon is also expensive, and has the danger of causing the hepatitis to recur. In addition, it is inconvenient because hospitalization may be required. Lamivudine is a nucleic acid derivative that shows a considerable efficacy with 40 to 70% of acute hepatitis patients, but its use is restricted to acute chronic hepatitis patients. Furthermore, viruses having resistance to lamivudine appear quickly, at the rate of approximately 20 to 30% within a year, and about 40% within two years.

Therefore, when an effective therapeutic vaccine is available, it will be cost-effective and convenient to administer. The possibility of using the presently available preventive vaccine for treatment of chronic HBV carriers has been tested in a small-scale human clinical trial. From this study, M. L. Michel et al (Vaccine 19, 2395-2399, 2001) demonstrated the possibility of using a vaccine for treatment of chronic HBV carriers, but their conclusion was that a stronger vaccine is required in order to treat efficiently. One other interesting observation is that the Pasteur-Merieux vaccine containing pre-S2 and S antigen is slightly better than the one containing only the S antigen. This study also suggests that inclusion of various antigens, e.g. pre-S1, pre-S2, and S, will improve the vaccine and that a therapeutic vaccine may need a strong adjuvant that can induce both strong humoral and cell-mediated immunity.

### SUMMARY OF THE INVENTION

It is an object of the invention to provide a pre-S protein that can be used as a component of HBV. vaccine and as an adjuvant.

Another object of the present invention is to provide a recombinant vector that can efficiently express pre-S gene of HBV in a yeast expression system.

Still another object of the present invention is to provide a transformant that secretes the pre-S protein of HBV into the culture media.

A still further object of the present invention is to provide formulation of a preventive and therapeutic vaccine including the recombinant pre-S protein.

A still further object of the present invention is to provide a diagnostic reagent that can detect antibodies against HBV in human or animal sera.

In order to achieve these objects, the present invention provides a modified pre-S of HBV which is either not glycosylated or is partially glycosylated. The modified pre-S includes a mutant pre-S in which one or both asparagines of wild-type pre-S at amino acid position 15 or 123 are substituted by any amino acids selected from the group consisting of alanine, arginine, cysteine, glutamine, glutamic acid, glycine, histidine, isoleucine, leucine, lysine, methionine, phenylalanine, proline, serine, threonine, tryptophan, tyrosine, and valine.

The present invention also provides a mutated pre-S gene encoding a modified pre-S of HBV of the present invention.

The present invention also provides a recombinant vector comprising:
(a) a promoter;
(b) a mutant pre-S gene of HBV; and
(c) a transcriptional termination factor.

The present invention also provides a transformant generated by introducing the recombinant vector of the present invention.

The present invention also provides an adjuvant comprising a pre-S of HBV. The pre-S can be prepared as a recombinant pre-S protein produced from the transformant.

The present invention also provides a hepatitis B virus vaccine comprising the modified pre-S.

The present invention also provides a diagnostic composition for detecting antibodies against the hepatitis B virus, hepatitis B virus surface antigens, or antigens coded by the pre-S, wherein the diagnostic composition comprises a modified hepatitis B virus pre-S.

The present invention also provides a producing method of pre-S of HBV, comprising:
(a) inserting the gene encoding the modified pre -S into a vector;
(b) transfecting the vector harboring the pre-S gene to a host cell; and
(c) producing the pre-S by culturing a transformant in media.

The present invention also provides a method of enhancing an antibody response to an antigen derived from HBV, wherein the method comprises administering an antibody-enhancing effective amount of an adjuvant of the present invention to a mammal or a bird.

The present invention also provides the use of a modified pre-S of HBV for preparing a vaccine for generating immunity for the HBV.

### BRIEF DESCRIPTION OF THE DRAWINGS

Fig. 1 is a diagram of the pIL20-pre-S vector according to the present invention;
Fig. 2 shows verification of the recombinant pre-S protein by SDS-PAGE and Western blot analyses carried out on the *Saccharomyces cerevisiae* 2805/pIL20-pre S culture medium using monoclonal anti-pre-S antibodies;
Fig. 3 shows verification of the recombinant pre-S protein by SDS-PAGE and Western blot analyses carried out on the *Saccharomyces cerevisiae* 2805/pIL20-pre S(dm) culture medium using monoclonal anti-pre-S antibodies;
Fig. 4 shows SDS-PAGE and Western blot analyses of the recombinant pre-S protein purified according to the present invention;
Fig. 5 shows SDS-PAGE and Western blot analyses of the recombinant pre-S protein (glycosylated and non- glycosylated form) purified according to the present invention;
Fig. 6 is a graph showing the recombinant pre-S protein analyzed by HPLC;
Fig. 7 shows a result of measuring immunogenicity in mice with the hepatitis B virus vaccine according to the present invention;
Fig. 8 shows a result of measuring the immunogenicity in a rat with the hepatitis B virus vaccine according to the present invention;
Fig. 9 shows a result of measuring immunogenicity in a rabbit with the hepatitis B virus vaccine according to the present invention;
Fig. 10 shows a result of measuring immune responses against S antigen in mice injected with the S antigen and pre-S protein of the HBV according to the present invention;
Fig. 11 shows a result of measuring IgG subtypes against S antigen in mice injected with the S antigen and a pre-S protein of the HBV according to the present invention; and
Fig. 12 shows a result of detecting HBV antibodies using a recombinant pre-S according to the present invention.

### DETAILED DESCRIPTION OF THE PREFERRED EMBODIMENTS

The term "pre-S gene" as used herein refers to a polynucleotide consisting of an HBV pre-S1 gene and an HBV pre-S2 gene, and "pre-S" refers to the protein encoded by the pre- S gene. The term "S antigen" refers to a polypeptide encoded by the S region of an HBV genome.

The term "HBV" means any subtypes of the virus, particularly adw, ayw, adr, ayr, and so on. The pre-S gene in this invention is preferably a pre-S gene of all subtypes of the hepatitis B virus, more preferably an adr subtype (SEQ ID NO: 1), an ayw subtype (SEQ ID NO: 2), an adw subtype (SEQ ID NO: 3), an adw2 subtype, and an adyw subtype, and most preferably a pre -S gene of an adr subtype and a pre-S gene of an ayw subtype.

The pre-S of the present invention is partially glycosylated, or is not glycosylated. The partially or non-glycosylated pre-S is a modified pre-S produced from cell transformed with gene coding a mutant pre-S. Also, the non-glycosylated pre-S can be synthesized by assembling individual amino acids by chemical means.

The pre-S produced from *Saccharomyces* is N-glycosylated on asparagines at amino acid positions 15 and 123. Therefore, the present invention provides a mutant pre-S which is generated by substituting the asparagines(s) at amino acid position 15 or 123 with amino acids selected from the group consisting of alanine, arginine, cysteine, glutamine, glutamic acid, glycine, histidine, isoleucine, leucine, lysine, methionine, phenylalanine, proline, serine, threonine, tryptophan, tyrosine, and valine. A more preferable amino acid is histidine or glutamine.

A preferred mutant pre-S includes:
(1) Pre-S-15m (SEQ ID NO:9), wherein asparagine at amino acid position 15 of the wild-type pre-S is substituted by histidine;
(2) Pre-S-123m (SEQ ID NO:10), wherein asparagine at amino acid position 123 of the wild-type pre-S is substituted by histidine; and
(3) Pre-S-dm (SEQ ID NO:11), wherein both asparagines at amino acid positions 15 and 123 of the wild-type pre-S are substituted by histidines.

The mutant pre-S of the present invention can be partially glycosylated when the pre-S is produced by expression vectors.

The present invention provides a mutated pre-S gene encoding the mutant pre-S of the present invention. The mutated pre-S gene has a mutated polynucleotide sequence encompassing the entire pre-S1 and pre-S2 coding regions. The mutated pre-S gene encodes pre-S that is partially glycosylated or not glycosylated.

The present invention also provides a recombinant vector including a promoter, a pre-S gene, and a transcription terminator. The pre-S gene is preferably a wild-type pre-S gene or a mutated pre-S gene. The recombinant vector is preferably used for transformation of a host cell to construct a transformant producing pre-S.

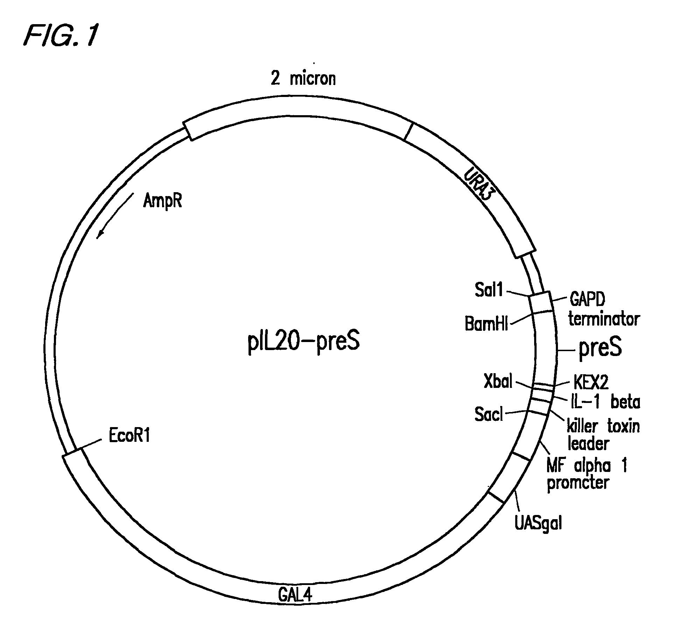

Examples of the recombinant vector are pIL20 -pre-S (adr) and pIL20-pre-S (ayw). The recombinant vector plL20-pre-S(adr) includes a pre-S gene of the adr subtype, and pIL20-pre-S (ayw) includes a pre-S gene of the ayw subtype of HBV. A map of the pIL20-pre-S vector is shown in the Fig. 1. The specific construction of the pIL20-pre-S vector is shown in the following Table 1.

**TABLE 1**

| Operating portion | Characteristics |
|---|---|
| Promoter | MF alpha I promoter |
| Secreting signal | Killer toxin, IL-1 beta leader |
| Cutting area | KEX2 |
| Insertion gene | Pre-S gene (coding region for pre-S1 and -S2) |
| Transcription Terminator | GAPD terminator |
| ura gene | URA3 |

Furthermore, the present invention provides a transformant producing a wild type pre-S of HBV or a mutant pre-S. The transformant is prepared by transfecting the recombinant vector including the pre-S gene of HBV to a host cell. The host cell is preferably yeast, and is more preferably selected from the group consisting of *Saccharomyces serevisiae, Pichia pastoris,* and *Yarrowia lipolytica.* The most preferred transformants are *Saccharomyces cerevisiae* 2805/pIL20-pre-S (adr), *Saccharomyces cerevisiae* 2805/pIL20-pre-S (ayw), or *Saccharomyces cerevisiae* 2805/pIL20-pre-dm. The *Saccharomyces cerevisiae* 2805/pIL20-pre-S (adr) produces the wild-type pre-S of SEQ ID NO: 4, and *Saccharomyces cerevisiae* 2805/pIL20-pre-S (ayw) produces the wild-type pre-S of SEQ ID NO: 5.

The transformants can be cultured at 30 °C for 24 hours using a sequential batch fermentation culture (1% yeast extract, 2% peptone, 2% dextrose).

In addition, the present invention provides a recombinant pre -S produced from the transformants. For producing the recombinant pre-S, the transformants can be cultured at 30 °C for 24 to 48 hours in a fed-batch culture media (1% yeast extract, 2% peptone) containing 2% galactose as a carbon source. The produced recombinant pre-S is secreted into the culture medium at up to 850 mg of recombinant pre-S per liter of culture media.

The recombinant pre-S can be purified by removing the yeast cell from the culture media, concentrating the cell-free culture media, dialyzing by using an ultra-fine membrane, and separating by ion exchange and with a molecular size separation columns.

Examples of the recombinant pre-S are a protein of SEQ ID NO:4, a protein of SEQ ID NO:5, a protein of SEQ ID NO:6, a Pre-S-15m of SEQ ID NO:9, a Pre-S-123m of SEQ ID NO:10 and a Pre-S-dm of SEQ ID NO:11.

The recombinant pre-S of the invention is partially glycosylated, or not glycosylated. The partially glycosylated recombinant pre-S can be digested with glycosidase to thereby obtain the non-glycosylated pre-S.

The present invention also includes use of the pre-S of the invention as an adjuvant. The adjuvant can be used for enhancing an immune response against a n antigen. The antigens are those derived from parasites or other pathogenic agents, which infect animals or birds, and more particularly, humans or livestock. Examples of parasites and pathogenic agents are prokaryotic organisms ; viruses including HIV, HBV, HCV, and rotavirus; and eukaryotic organisms such as yeast and fungi, protozoa, metazoa, and amoebae.

The adjuvant contains the pre-S or the recombinant pre-S of the present invention, i.e., a partially glycosylated pre-S or non-glyposylated pre-S.

The present invention provides use of the pre-S of the invention for preparing an adjuvant enhancing an immune response to an antigen derived from HBV by administering an antibody-enhancing effective amount of the adjuvant to humans, mammals, or birds. The mammals include livestock animals, laboratory animals, domestic animals , and captive wild animals; and the birds include chickens or any other domestic or wild birds.

Also, the present invention provides a vaccine that can prevent HBV infection or treat chronic HBV carriers. The HBV vaccine contains the pre-S or the recombinant pre-S of the present invention, and further contains the S antigen. The vaccine including the pre-S and the S antigen can effectively prevent and treat acute hepatitis or chronic hepatitis caused by the HBV. The HBV vaccine can include S antigens and pre-S at a weight ratio ranging from 10:1 to 1:10.

The HBV vaccine can be prepared by the common method of preparing vaccines, and may further includes adjuvant, or one or more acceptable pharmacological compositions. The pharmacological compositions are vehicles or diluents. Examples of the vehicle or the diluents are saline, buffered saline, dextrose, water, glycerol , and ethanol, but they are not limited thereto. An example of the adjuvant is aluminum hydroxide, and the adjuvant used can be any one available.

The HBV vaccine of the present invention can be administered orally or parenterally, preferably being used in an injectable form, and the vaccine can be in the form of suspension or solution. The dosage of the HBV vaccine is one commonly used for vaccination using the general HBV vaccine and it is desirable that the exact dosage is applied differently, depending on the condition, weight, and age of patients, etc.

The HBV vaccine of the present invention can be used for generating immunity for HBV.

Furthermore, the present invention provides a diagnostic composition that can determine the formation of antibodies against HBV and detect antibodies against HBV, HBV surface antigens, or antigens coded by the pre-S gene. The diagnostic composition contains the pre-S of the present invention as the HBV specific antibody-capturing antigen for the Enzyme-Linked Immunosorbent Assay ("ELISA") method, or for various formats of point of care tests.

Hereinafter, preferred examples are provided to assist the understanding of the present invention. The following examples, however, are only for the purpose of facilitating the understanding of the invention and should not be construed to be limiting in any sense.

### EXAMPLE 1.

### Generation of yeast cell line expressing recombinant pre-S

### (1) Production of pIL20-pre-S vector

The structure of the recombinant vector is schematically illustrated in Fig. 1.

To clone the pre-S gene, Korean variant HBV 315 (base sequence Accession No. AF286594) was selected as the main type among the adr subtypes of the hepatitis B virus, and the pre-S gene was amplified by the PCR method. The primers used were a sense primer of SEQ ID NO: 7 and an antisense primer of SEQ ID NO: 8. The sense primer binds at position 2848 of the HBV gene, and it includes KEX 2 and Xbal restriction sites. Furthermore, the antisense primer is specific at the position 130, and includes a terminal codon (TAG) and a *BamHI* restriction site.

PCR was carried out over thirty cycles under the condition of denaturation (94 °C , 1 min), annealing (45 °C, 1 min), and polymerization (72 °C, 1 min), resulting in a 522 bp PCR product of SEQ ID NO: 1. The PCR product was digested with the restriction enzymes *X*bal and *BamH*I*,* rendering it with cohesive ends. The PCR product was inserted into the pIL20 vector digested with the same enzymes, and pIL20-pre-S (adr) was constructed.

### (2) Production of pIL20-pre-S (ayw)

The HBV ayw subtype (accession No. X02496) was used as a template, and a pIL20-pre-S (ayw) vector was constructed.

### (3) Production of the transformants

The pIL20-pre-S vector (adr) or pIL20-pre-S (ayw) vector was transformed to yeast *(Saccharomyces cerevisiae* 2805) by a conventional transformation method. The S. *cerevisiae* 2805 is a ura host having the genotype Matapep4::HIS3prb1 -1.6Rcan1GAL2his3- 200ura3-52.

The transformant/pIL20-pre-S (adr) or /pIL20-pre-S (ayw) was selected by culturing on a ura⁻, ade⁻, and trp⁻ selection medium (CAA-glucose).

The yeast transformed by the pIL20-pre-S (adr) vector was named *Saccharomyces cerevisiae* 2805/pIL20-pre-S(adr), and was deposited on April 16, 2001 at the Korean Collection for Type Cultures ("KCTC"), which is an international deposit authority, under KCTC 0987BP. The yeast transformed by pIL20-pre-S (ayw) vector was named *Saccharomyces cerevisiae* 2805/pIL20-pre-S (ayw), and was deposited on May 8, 2001 at the Korean Collection for Type Cultures ("KCTC") under KCTC 1004BP.

### EXAMPLE 2.

### Generation of yeast cell line expressing recombin ant mutant pre-S

To increase the immunogenicity of the recombinant pre -S, glycosylation sites of the pre-S region (amino acids 15 and 123 Asparagine) were mutated into histidine or glutamine by site-directed mutagenesis using plasmid pIL20 -pre-S as a template.

The nucleotide codon AAT encoding asparagine at the amino acid #15 was mutated into the codon CAC encoding histidine, and pIL20 -pre-S (15m) was constructed. The nucleotide codon AAC encoding asparagine at the amino acid #123 was mutated into the codon CAC encoding histidine, and pIL20-pre-S (123m) was constructed. Both codons encoding asparagine at the amino acid #15 and #123 were mutated into the nucleotide codon CAC encoding histidine, and the double mutant plasmid pIL20-pre-S (dm) was constructed.

The pIL20-pre-S (15m), pIL20-pre-S (123m), or pIL20-pre-S (dm) was again used to transform yeast, *Saccharomyces cerevisiae* 2805. Through the same screening process described above, the colony having the highest expression rate of the recombinant protein was selected as a cell line for production to establish a master cell bank.

### Example 3

### Production of the recombinant pre-S

### (1) Confirmation of the recombinant pre-S production

The transformants *(Saccharomyces cerevisiae* 2805/pIL20-pre-S (adr), *Saccharomyces cerevisiae* 2805/pIL20-pre-S (ayw), and *Saccharomyces cerevisiae* 2805/ pIL20-pre-S (dm) were respectively cultivated in a sequential batch fermentation culture (1 % yeast extract, 2% peptone, 2% glucose) at 30 °C for about 24 hours. When the OD level at 600 nm reached 20 to 30, a fed-batch culture medium (1% yeast extract, 2% peptone) containing 2% galactose, which is an inducer of pre-S gene expression and the only carbon source in the induction media, was added slowly, and then cult ivation was continued for about 24 hours to induce the expression of the pre-S.

20 to 30 ul of the culture media were subjected to an SDS-PAGE analysis, and a Western blot analysis was carried out with a pre-S specific monoclonal anti-pre-S antibody to confirm the expression of the pre -S.

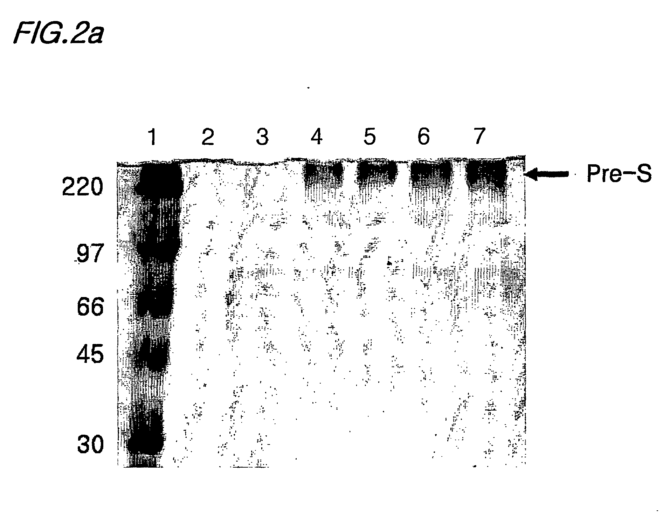

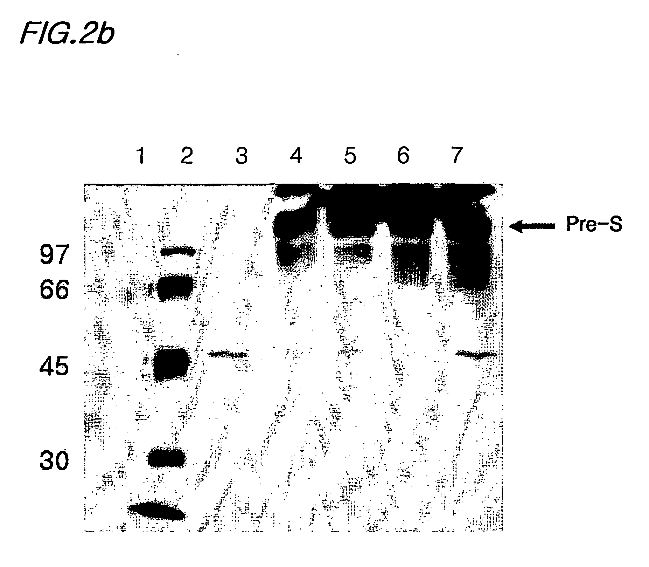

Fig. 2a is a picture of an SDS-PAGE analysis of the culture media, verifying pre-S expressed in *Saccharomyces cerevisiae* 2805/pIL20-pre-S, and Fig. 2b shows a result of the Western blot analysis of the electrophoresis gel shown in the Fig. 2a. Lanes 1 and 2 of Fig. 2 indicate the size of the protein markers, lane 3 is the culture media of *Saccharomyces cerevisiae* 2805 transformed with the pIL20 vector without the pre-S gene, lanes 4 and 5 are the culture medium of *Saccharomyces cerevisiae* 2805/plL20-pre-S(adr), and lanes 6 and 7 are the culture media of *Saccharomyces cerevisiae* 2805/pIL20-pre-S (ayw). The recombinant pre-S expressed from the transformant is extensively glycosylated so that the SDS-PAGE analysis shows a broad band with a molecular size ranging from 150 to 250 kDa.

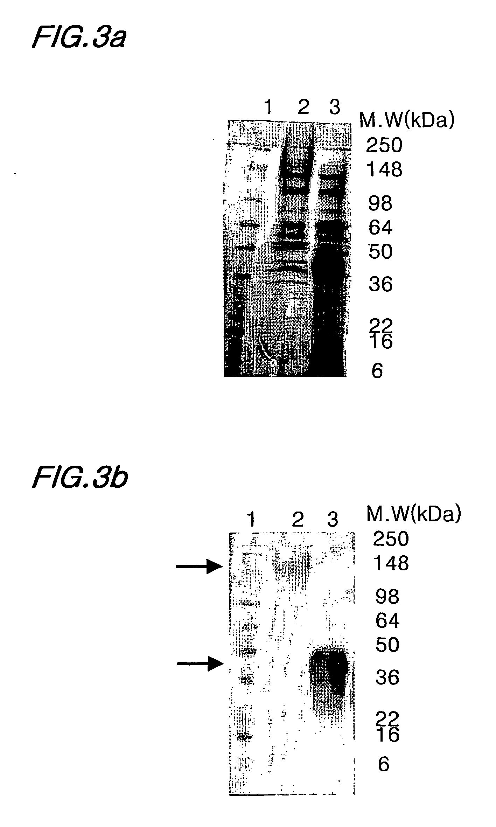

Figs. 3a and 3b show SDS-PAGE (a) and Western bolt (b) analyses conducted on the *Saccharomyces cerevisiae* 2805/ pIL20-pre-S (dm) culture media using a monoclonal anti-pre-S antibody. Lane 1 is a size maker of protein, lane 2 is a wild-type pre-S, and lane 3 is a culture media of *Saccharomyces cerevisiae* 2805/ pIL20-pre-S (dm). The recombinant mutant pre-S, Pre-S-dm expressed from *Saccharomyces cerevisiae* 2805/ pIL20-pre-S (dm) was partially glycosylated, and it showed a molecular size of about 50 kDa .

### (2) Fermentation

The recombinant yeast producing pre-S was cultivated in a 10 L fermentation jar. Seed cultures were established in a basic culture media (0.6% casamino acid, 2% glucose, 1 x yeast nitrogen base) using 25 ml and 500 ml flasks.

The exhaustion of the glucose was verified with a glucose assay kit while culturing for 36 hours in a fed-batch fermentation culture. Then, the media (1% yeast extract, 2% peptone, 2% galactose) was added slowly and cultivated for 36 hours to induce the expression of the pre- S. The density of the cell was an OD level of about 50 at 600 nm and the concentrat ion of the pre-S was between 50 and 60 mg/L.

### (3) Purification

### 3-1. Collection and filtering stage

The recombinant pre-S expressed in the transformed yeast was secreted into the culture media. The cells were removed from the culture media by centrifugation, and then the culture solution was fine-filtered several times through a Durapore membrane with a 0.45um size.

### 3-2. Ultra-fine filtering stage

The above culture solution was subjected to ultrafiltration and diafiltration through an ultra-fine filter with a molecular weight cut-off value of 30 kDa.

### 3-3. Ion-exchange chromatography

The filtrate was loaded onto the SP-sepharose FF column equilibrated with 25 mM of an acetate buffer solution (pH 4.5), and the column was washed with the same buffer solution followed by eluting with a sodium chloride concentration gradient (0.2 M NaCl).

### 3-4. Differential size separation stage

The eluents separated by the above Ion-exchange chromatography were again concentrated through the ultra- fine filter and then loaded onto a sephacryl S-300 (or S-200) gel filtration column. Then, the Phosphate Buffered Saline (PBS, pH 7.0) was passed through the column to elute pre -S.

Among the eluted fractions, the fractions containing the recombinant pre-S [Kav = 0.440 - 0.475, Kav = Ve-Vo/Vt - Vo, Ve: eluted volume of the protein, Vo: eluted volume of blue dextran, Vt: total volume of the column] were pooled and passed through a 0.22 mm filter, and then stored in a freezer.

The following Table 2 shows the purification yield.

**TABLE 2**

| Step | Total protein | Total pre-S | Specific activity (mg pre-S/ mg protein) | Purification yield | Purity (Fold) |
|---|---|---|---|---|---|
| Filtering stage | 309,440 mg | 347.8 mg | 0.00112 | 100% | 1 |
| Ultra-filtering stage | 14,780 mg | 336.4 mg | 0.02276 | 96.7% | 20.3 |
| Ion-exchange chromatography | 360 mg | 235.5 mg | 0.65417 | 67.7% | 584.1 |
| Size difference separation | 157.9 mg | 155.43 mg | 0.98486 | 44.7% | 878.9 |

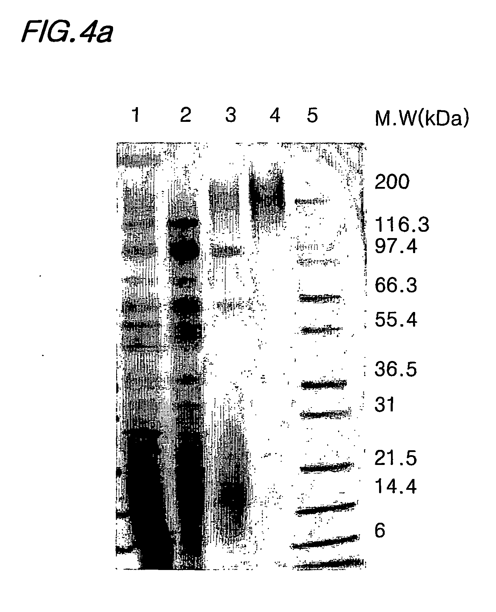

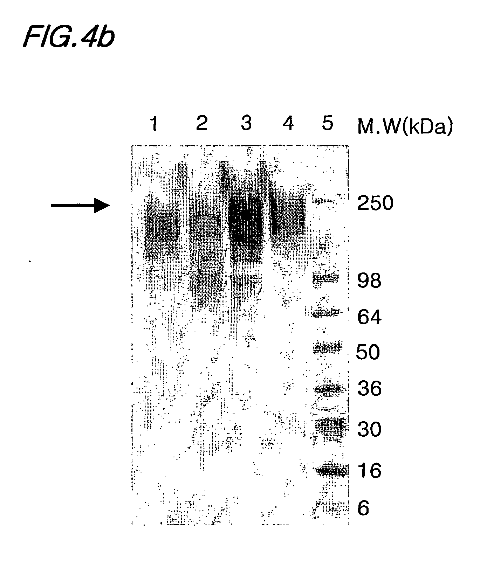

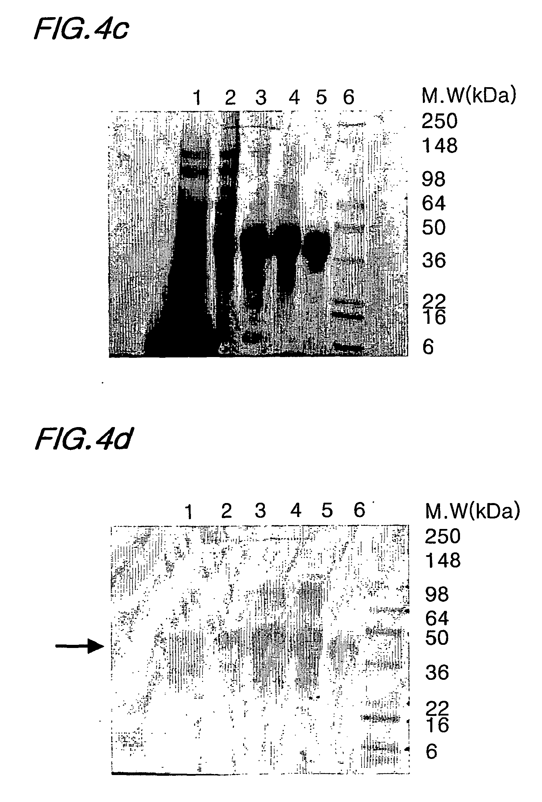

Figs. 4a to 4d shows SDS-PAGE and Western blot analyses of samples purified from each step of the pu rification process. Fig. 4a shows an SDS-PAGE analysis conducted on culture media of *Saccharomyces cerevisiae* 2805/pIL20-pre-S (adr), and 4b is Western blot analysis of the same. Fig 4c is an SDS-PAGE analysis, and 4d is a Western blot analysis conducted on culture media of *Saccharomyces cerevisiae* 2805/ pIL20-pre-S (dm). In Figs. 4a and 4b, lane 1 is a culture media, lane 2 is a sample purified in the filtering stage, lane 3 is a sample purified in the ion-exchange chromatography stage (Sp-Sepharose), lane 4 is a sample purified in the differential size separation stage (S300), and lane 5 is a size maker. In Figs. 4c and 4d, lane 1 is a culture media, lane 2 is a sample purified in the filtering stage, lane 3 is a sample purified in the ion-exchange chromatography stage (Sp-Sepharose), lane 4 is a sample purified in the ion-exchange chromatography stage (Q-Sepharose), lane 5 is a sample purified in the differential size separation stage (S200), and lane 6 is a size maker.

*Saccharomyces cerevisiae* 2805/pIL20-pre-S(adr) expressed a hyperglycosylated recombinant pre-S, and *Saccharomyces cerevisiae* 2805/pIL20-pre-S (dm) expressed a partially glycosylated Pre-S-dm. A recombinant pre-S with a high degree of purity can therefore be obtained though the purification process.

### EXAMPLE 4.

### The physical and chemical characteristics of the recombinant pre-S

To find out whether the recombinant pre-S was glycosylated or not, the recombinant pre-S produced by *Saccharomyces cerevisiae* 2805/ pIL20-pre-S (adr) was digested with N-glycosidase F (Calbiochem), and Western blot analysis was conducted.

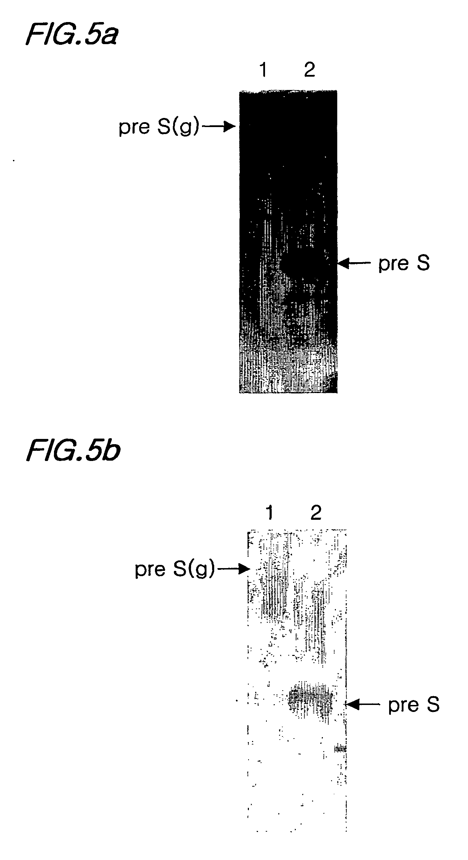

Figs. 5a and 5b show SDS-PAGE (a) and Western blot (b) analyses of the purified recombinant pre-S according to the present invention. Lane 1 is the purified recombinant pre-S, and lane 2 is the recombinant pre-S treated with N-glycosidase F. After removal of the glycosyl moiety, the molecular weight of the recombinant pre-S was observed to be approximately 20 kDa.

To verify the purity of the recombinant pre-S of Example 3, HPLC analysis was carried out. TSK G3000SW was used as the column, and the flow rate was 1 ml/min on the PBS and it was detected at 214 nm.

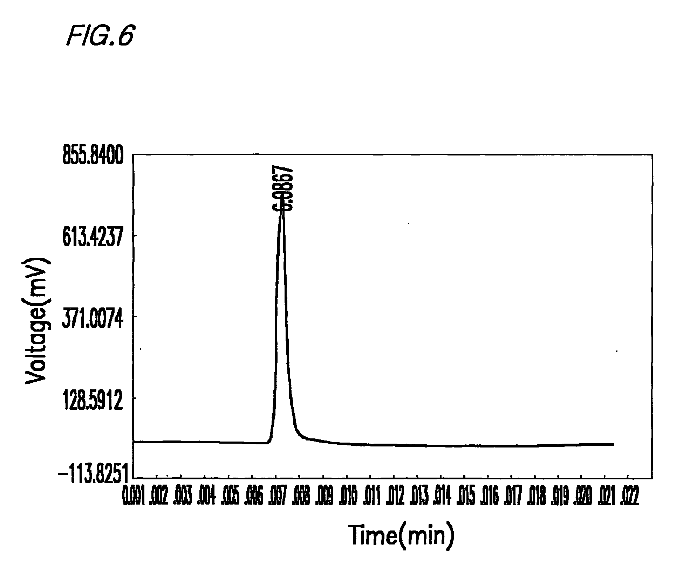

Fig. 6 shows the HPLC chart of the recombinant pre-S. A single peak on the graph confirms the high purity of the recombinant pre -S, and there is no indication of contamination with any other materials.

In addition, the N-terminal amino acid sequence of the recombinant pre-S purified in the above Example 3 was analyzed in a protein sequence analysis system (Procise cLC 492, Applied Biosystems), and it was compared with the amino acid sequence deduced from the sequence of the pre-S gene.

Table 3 shows an amino acid sequence of recombinant pre-S and the amino acid sequence deduced from the base sequence of the pre-S gene. The "ND" in Table 3 means "not determined". They matched perfectly, except for one.

**TABLE 3**

| | 1 | 2 | 3 | 4 | 5 | 6 | 7 | 8 | 9 | 10 | 11 | 12 | 13 | 14 |
|---|---|---|---|---|---|---|---|---|---|---|---|---|---|---|
| Recombinant pre-S | Met | Gly | Gly | Trp | Ser | Ser | Lys | Pro | ND | Gln | Gly | Met | Gly | Thr |
| Derived Amino acid | Met | Gly | Gly | Trp | Ser | Ser | Lys | Pro | Arg | Gln | Gly | Met | Gly | Thr |

### EXAMPLE 5.

### Verification of the Recombinant Pre-S by Monoclonal Anti-pre S.

The specific antigen-antibody reactivity of the recombinant pre-S was examined. The recombinant pre-S produced by *Saccharomyces cerevisiae* 2805/ pIL20-pre-S (adr) was digested with N-glycosidase F (Calbiochem), and Western blot analysis was conducted.

The recombinant pre-S reacted with the monoclonal anti-pre-S2 antibody showed a size ranging from 150 to 250 kDa, and it showed at approximately 20 kDa after removing the glucose moiety. Therefore, the recombinant pre-S maintains the specificity of antigen antibody reactivity independent of glycosylation.

### EXAMPLE 6.

### Preparation of HBV vaccine

10 ug of the recombinant pre-S of Example 3 were absorbed in 0.8 mg of aluminum hydroxide to formulate a vaccine. It is possible to adsorb up to 20 ug of pre S with 0.8 mg Alum.

### EXAMPLE 7.

### Preparation of HBV vaccine

5-20 ug of the recombinant pre-S of Example 3 were used for the vaccine formulation.

### EXAMPLE 8.

### Preparation of HBV vaccine

1-200 ug of the recombinant pre-S were mixed with complete Freud's adjuvant (CFA) to prepare immunogen for immunogenicity tests.

### EXAMPLE 9.

### Verification of the immunogenicity of the recombinant pre-S

### (1) Immunogenicity experiment- Mouse experiment

10 ug of pre S prepared by Examples 6, 7, and 8 were intramuscularly injected to each Balb-c mouse at intervals of 0, 2, and 4 weeks. 30 days after the first injection, blood was drawn to observe the formation of antibodies against the anti-recombinant pre -S by the ELISA method.

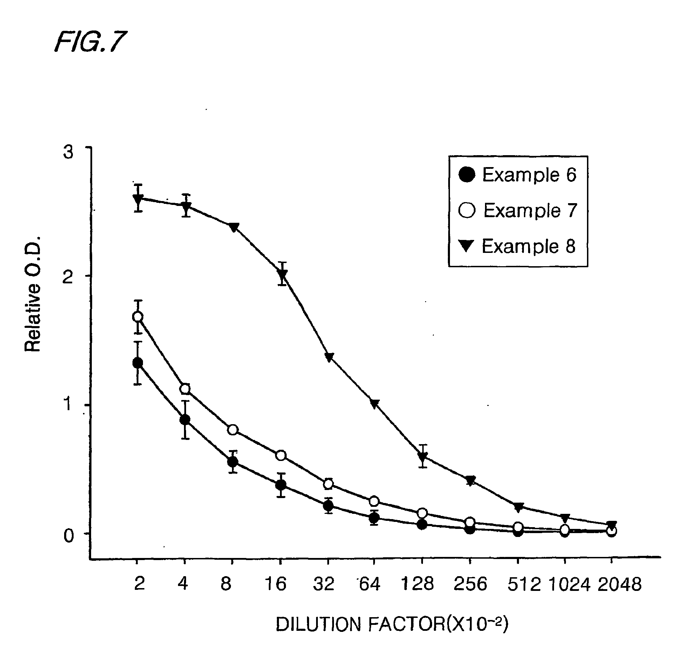

Fig. 7 shows results of measuring immunogenicity in mice with the recombinant pre-S of HBV. ● is an antibody titer induced by the vaccine of Example 6, ○ is an antibody titer induced by the vaccine of Example 7, and is an antibody titer induced by the vaccine of Example 8. As shown in Fig. 7, mice were immunized with the recombinant pre-S alone or by co-injection with an adjuvant, and the vaccine of Example 8 shows substantially increased immunogenicity when compared to vaccines of Examples 6 and 7.

### (2) Immunogenicity experiment-Rat experiment

The vaccine of Example 6 and a vaccine formulated by PAMIII, a lipopeptide proven to be harmless to a human body, were examined for immunogenicity in rats.

The vaccine of Example 8 was used as a positive control, and a composition without the recombinant pre-S was used as a negative control.

20 ug of vaccines (vaccine of Example 6, vaccine formulated by PAMIII, positive control, and negative control) were intramuscularly injected into Sprague Dawley rats at intervals of 0, 2, and 4 weeks respectively. 30 days after the first injection, antibodies to pre-S in sera of rats immunized with the vaccines were tested by the ELISA method.

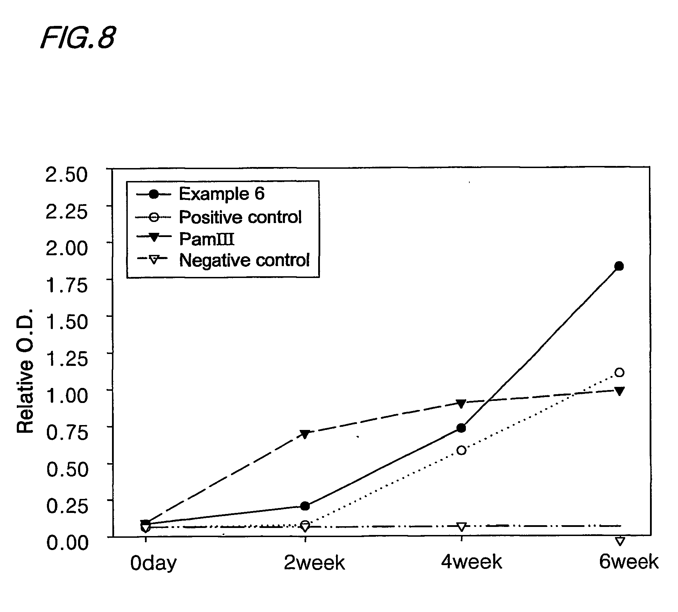

Fig. 8 shows results of measuring immunogenicity in rats with the recombinant pre-S of HBV. ● is an antibody titer induced by the vaccine of Example 6, ○ is an antibody titer induced by the positive control, ▼ is an antibody titer induced by the vaccine formulated with PAMIII, and ∇ is an antibody titer induced by the negative control. As can be seen in Fig. 8, the vaccine of Example 6 and the vaccine formulated with PAMIII induced an immune response against pre-S in rats. Furthermore, lipopeptide PAMIII induced antibodies against the pre-S antigens as well, which shows the potential of its use as an immune support agent.

### (3) Immunogenicity experiment- Rabbit experiment

To see immunogenicity of the recombinant pre-S, 100 ug of the recombinant pre-S was intramuscularly injected together with complete Freund' s adjuvant three times into rabbits at intervals of 0, 2, and 6 weeks. Ten days after the final injection, blood was drawn and sera were separated to assess the pre-S specific antibody titer, by the ELISA method.

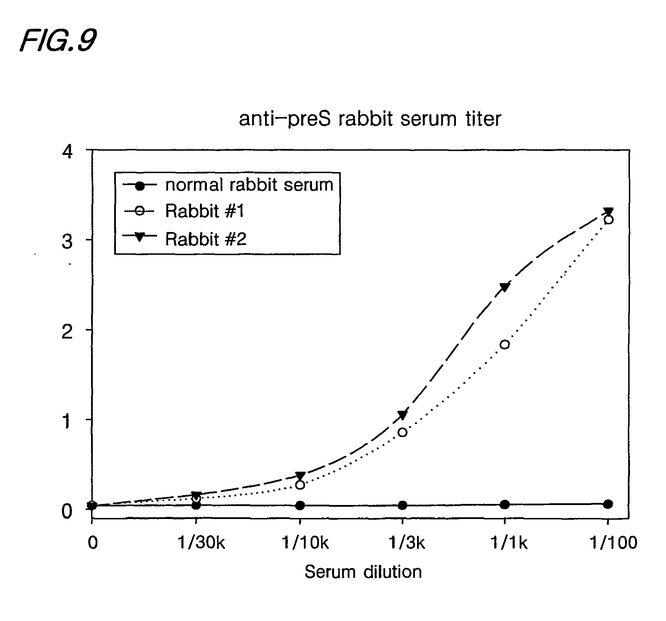

Fig. 9 shows a result of measuring immunogenicity in rabbits with the recombinant pre-S of HBV. ● is a normal rabbit sera, ○ is a sera of immunized rabbit #1, and ▼ is a sera of immunized rabbit #2.

The recombinant pre-S showed a high antibody titer in rabbits.

### (4) Immunogenicity of the modified recombinant pre-S

Similarly, immunogenicity of other modified pre-S, two single mutants (Pre-S-15m and Pre-S-123m), a double mutant (Pre-S-dm), and a double mutant linked to the tetanus toxoid sequence, was tested in Balb/c mice and ICR mice. All were highly immunogenic with complete Freund's adjuvant, but the antibody titers were very low with aluminum hydroxide as an adjuvant.

### EXAMPLE 10.

### Verification of the adjuvanticity of the modified recombinant pre-S

The modified recombinant pre-S was used for enhancing immune response.

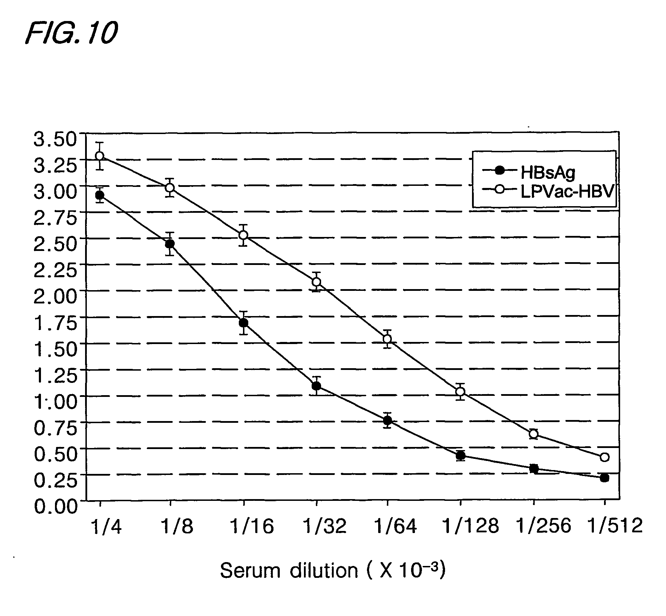

When a mixture (hereinafter referred to as "LPVac-HBV") of the modified recombinant pre-S (Pre-S-dm) and the S antigen is adsorbed to aluminum hydroxide (alum) and administered by intraperitonial injection, the immunogenicity of LPVac-HBV was increased several-fold over the S antigen ("HBsAg") alone (Fig. 10).

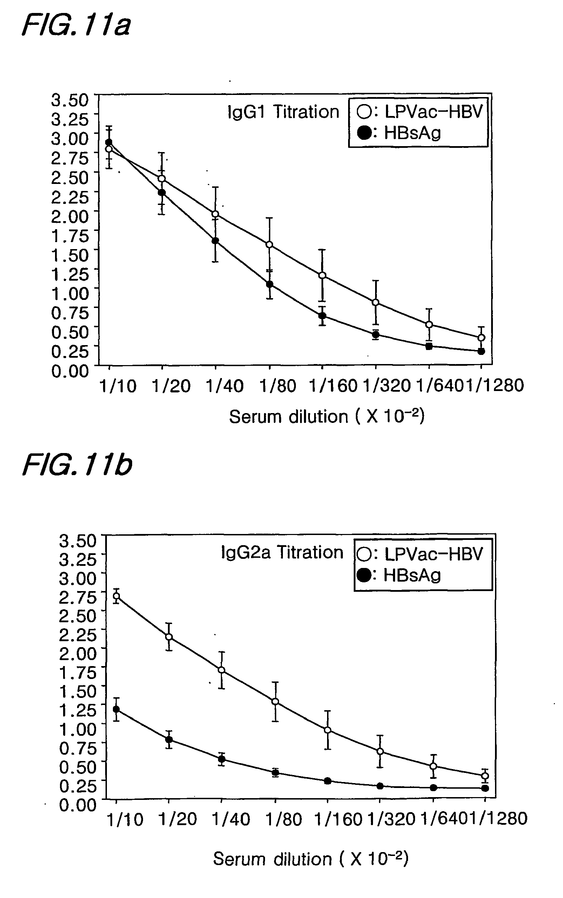

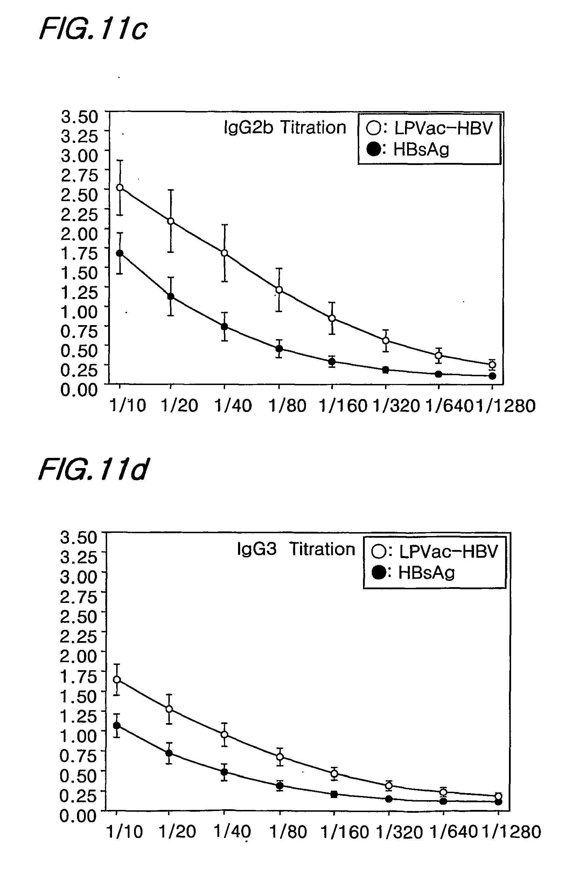

The sera were obtained from the mice immunized against LPVac-HBV or HBsAg, and IgG subtypes were also determined (Fig. 11). The IgG2a was increased by about 10-fold over the S antigen alone.

Therefore, pre-S could be included in a preventive vaccine formulation to improve the overall quality of currently available vaccines containing only S antigen, and in therapeutic vaccine formulation.

### EXAMPLE 11.

### Toxicity test - single administered toxicity test (acute)

The vaccine of Example 6 was abdominally injected to ICR mice at 0, 0.125, 0.25, 0.5, 1, and 2 mg/kg (body weight). The body weight, clinical symptoms, and pathological findings of the testing group were observed and compared with the negative control group to which the vaccine was not injected. In this experimen, no unusual symptoms were observed. None of the mice died even under maximum dosage (2mg/kg); thus, LD₅₀ could not be determined. Therefore, it can be seen that the vaccine of Example 6 had no acute toxicity for the mice.

### EXAMPLE 12.

### Toxicity test - repeatedly administered toxicity test (subacute)

The vaccine of Example 6 was hypodermically injected at 0, 50, 100, and 200 ug/kg (body weight) five times per week to Sprague Dawley rats, then changes were observed. Compared to the control group to which the vaccine was not injected, the SD rats immunized with the vaccine of Example 6 did not show any changes in body weight, clinical symptoms, or pathology.

### EXAMPLE 13.

### Toxicity test - skin test

Fur of New Zealand white rabbits was removed in the size of 2.5 cm x 2.5 cm, one of which had the horny layer removed with a needle for hypodermic injection, and the other was left untouched. 0.5 mg of the vaccine of Example 6 was smeared onto surgery gauze and then placed on both areas to cover them. The control group was treated with PBS by the same procedure. After skin contact for 24, 48, and 72 hours, the treated parts were observed according to the Draize Scoring System to determine the safety of the medication.

Compared to the control group, the skin of the New Zealand white rabbits treated with the recombinant pre-S vaccine showed no clinical changes such as erythemas, eschars, and edemas.

### EXAMPLE 14.

### Use of pre-S as a diagnostic reagent

Use of the recombinant pre-S of Example 3 as an anti-pre-S antibody-capturing antigen was tested.

Ninety-six well plates (Nunc Maxicorp) were coated with 50 ng/well pre-S, according to a standard method. The coated plates were washed three times with PBST (137 mM NaCl, 2.7 mM KCI, 10 mM Na₂HPO₄, 2 mM KH₂PO₄) and then 100 ul of 1% Bovine Serum Albumin ("BSA") was added to each well to block remaining antigen binding sites on the plate. After one hour incubation at 37 °C, the plates were washed with PBS and stored at 4 °C until use.

In this assay, anti-pre-S2 antibodies prepared from mice or a human serum with the determined antibody titer were used as positive controls, and PBS was the negative control. The reaction volume of each well was 100 ul, and they underwent reaction for 90 minutes at 37 °C. In the same manner, 100 ul each of human serum and serially-diluted human serum were assayed for anti-pre-S antibodies. Goat anti-mouse IgG or goat anti-human IgG conjugated with horseradish peroxidase ("HRP") as a secondary antibody and then o-Phenylenediamine ("OPD") was added as a sub strate to develop color, at room temperature. After stopping the coloring reaction with 3M HCl, the OD level was measured at 492 nm.

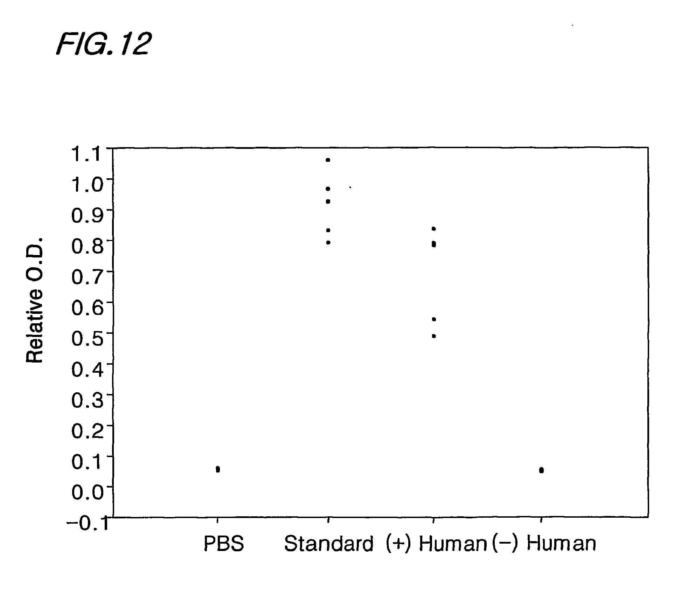

Fig. 12 shows that the recombinant pre S could capture the anti -pre S antibody in the HBV-positive human sera to the similar extent of the positive control (e.g. monoclonal pre S2 antibody). Therefore, the recombinant pre S can be used to develop diagnostic kit to detect the HBV -specific antibodies from the clinical sample.

## Claims

1. A non-glycosylated or partially glycosylated pre-S of hepatitis B virus (HBV) in which one or both asparagines at positions 15 and 123 of a wild-type pre-S are substituted by an amino acid selected from the group consisting of alanine, arginine, cysteine, glutamine, glutamic acid, glycine, histidine, isoleucine, leucine, lysine, methionine, phenylalanine, proline, serine, threonine, tryptophan, tyrosine, and valine.

2. The pre-S of HBV according to claim 1, wherein the wild-type pre-S is derived from an HBV selected from the group consisting of adr, ayw, adw, adw2, and adyw subtypes.

3. The pre-S of HBV according to claim 1, wherein the pre-S is selected from the group consisting of Pre-S-15m (SEQ ID NO:9), Pre-S-123m (SEQ ID NO:10), and Pre-S-dm (SEQ ID NO:11).

4. A pre-S gene encoding the pre-S of HBV of any one of claims 1 to 3.

5. A recombinant vector comprising:
(a) a promoter;
(b) a pre-S gene of claim 4; and
(c) a transcriptional terminator.

6. The recombinant vector according to claim 5, wherein the vector is pIL20-pre-S (adr) as deposited at the KCTC under accession number KCTC 0987BP or pIL20-pre-S (ayw) as deposited at the KCTC under accession number KCTC 1004BP.

7. A transformant comprising the recombinant vector of claim 5.

8. The transformant according to claim 7, wherein the transformant is yeast.

9. The transformant according to claim 7, wherein the transformant is selected from the group consisting of Saccharomyces cerevisiae 2805/pIL20-pre-S (KCTC 0987BP) and Saccharomyces cerevisiae 2805/pIL20-pre-S (ayw) (KCTC 1004BP).

10. A recombinant pre-S produced from the transformant of claim 7.

11. An adjuvant comprising a pre-S of HBV of any one of claims 1 to 3.

12. An HBV vaccine comprising a pre-S of HBV of any one of claims 1 to 3.

13. The HBV vaccine according to claim 12, wherein the HBV vaccine further comprises an S antigen of HBV.

14. A diagnostic composition for detecting antibodies against HBV, HBV surface antigens, or antigens coded by the pre-S gene, wherein the diagnostic composition comprises one selected from the group consisting of the pre-S of HBV of any one of claims 1 to 3.

15. A producing method of an HBV pre-S, comprising:
(a) inserting a pre-S gene of claim 4 into a vector;
(b) transfecting the vector harboring the pre-S gene of claim 4 to a host cell; and
(c) producing the pre-S by culturing a transformant in medium.

16. Use of a pre-S of HBV of any one of claims 1 to 3 for preparing an adjuvant enhancing an antibody against an antigen derived from HBV in a mammal or a bird.

17. Use according to claim 16, wherein the mammal is selected from the group consisting of a human, a livestock animal, a laboratory test animal, a domestic animal, and a captive wild animal.

18. Use according to claim 16, wherein the bird is a chicken or other poultry bird.

19. Use of a pre-S of HBV of any one of claims 1 to 3 for preparing a vaccine for generating immunity for the hepatitis B virus.

20. Use according to claim 19, wherein the HBV vaccine further comprises a HBV S antigen.

## Patentansprüche

1. Unglykosyliertes oder teilglykosyliertes pre-S von Hepatitis-B-Virus (HBV), in dem ein oder beide Asparagine an den Positionen 15 und 123 eines Wildtyp-pre-S durch eine Aminosäure substituiert sind, die ausgewählt ist aus der Gruppe bestehend aus Alanin, Arginin, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin.

2. HBV-pre-S nach Anspruch 1, wobei das Wildtyp-pre-S von einem HBV stammt, der ausgewählt ist aus der Gruppe bestehend aus adr-, ayw-, adw-, adw2- und adyw-Subtypen.

3. HBV-pre-S nach Anspruch 1, wobei das pre-S ausgewählt ist aus der Gruppe bestehend aus Pre-S-15m (SEQ ID NO:9), Pre-S-123m (SEQ ID NO:10) und Pre-S-dm (SEQ ID NO:11).

4. pre-S-Gen, welches das HBV-pre-S nach einem der Ansprüche 1 bis 3 codiert.

5. Rekombinanter Vektor, umfassend:
(a) einen Promotor;
(b) ein pre-S-Gen nach Anspruch 4; und
(c) einen Transkriptionsterminator.

6. Rekombinanter Vektor nach Anspruch 5, wobei der Vektor pIL20-pre-S (adr) ist, wie hinterlegt bei der KCTC unter der Zugangsnummer KCTC 0987BP, oder pIL20-pre-S (ayw) ist, wie hinterlegt bei der KCTC unter der Zugangsnummer KCTC 1004BP.

7. Transformante, umfassend den rekombinanten Vektor nach Anspruch 5.

8. Transformante nach Anspruch 7, wobei die Transformante Hefe ist.

9. Transformante nach Anspruch 7, wobei die Transformante ausgewählt ist aus der Gruppe bestehend aus Saccharomyces cerevisiae 2805/pIL20-pre-S (KCTC 0987BP) und Saccharomyces cerevisiae 2805/pIL20-pre-S (ayw) (KCTC 1004BP).

10. Rekombinantes pre-S, produziert von der Transformante nach Anspruch 7.

11. Adjuvans, umfassend ein HBV-pre-S nach einem der Ansprüche 1 bis 3.

12. HBV-Impfstoff, umfassend ein HBV-pre-S nach einem der Ansprüche 1 bis 3.

13. HBV-Impfstoff nach Anspruch 12, wobei der HBV-Impfstoff weiterhin ein HBV-S-Antigen umfasst.

14. Diagnostische Zusammensetzung zum Nachweis von Antikörpern gegen HBV, HBV-Oberflächenantigenen oder Antigenen, die durch das pre-S-Gen codiert werden, wobei die diagnostische Zusammensetzung eines ausgewählt aus der Gruppe bestehend aus dem HBV-pre-S nach einem der Ansprüche 1 bis 3 umfasst.

15. Verfahren zur Herstellung von HBV-pre-S, umfassend:
(a) Insertieren eines pre-S-Gens nach Anspruch 4 in einen Vektor;
(b) Transfizieren des Vektors, der das pre-S-Gen nach Anspruch 4 enthält, in eine Wirtszelle; und
(c) Produzieren des pre-S durch Kultivieren einer Transformanten in Medium.

16. Verwendung eines HBV-pre-S nach einem der Ansprüche 1 bis 3, zur Herstellung eines Adjuvans, das in einem Säuger oder einem Vogel die Bildung eines Antikörpers gegen ein Antigen fördert, das von HBV stammt.

17. Verwendung nach Anspruch 16, wobei der Säuger ausgewählt ist aus der Gruppe bestehend aus einem Menschen, einem Nutztier, einem Laborversuchstier, einem Haustier und einem in Gefangenschaft lebenden Wildtier.

18. Verwendung nach Anspruch 16, wobei der Vogel ein Huhn oder ein anderes Geflügeltier ist.

19. Verwendung eines HBV-pre-S nach einem der Ansprüche 1 bis 3 zur Herstellung eines Impfstoffs zur Immunitätserzeugung gegen den Hepatitis-B-Virus.

20. Verwendung nach Anspruch 19, wobei der HBV-Impfstoff weiterhin ein HBV-S-Antigen umfasst.

## Revendications

1. Protéine pré-S non glycosylée ou partiellement glycosylée du virus de l'hépatite B (VHB) dans laquelle l'une ou les deux asparagines en positions 15 et 123 d'une protéine pré-S de type sauvage sont substitués par un acide aminé choisi dans le groupe constitué d'alanine, d'arginine, de cystéine, de glutamine, d'acide glutamique, de glycine, d'histidine, d'isoleucine, de leucine, de lysine, de méthionine, de phénylalanine, de proline, de sérine, de thréonine, de tryptophane, de tyrosine et de valine.

2. Protéine pré-S du VHB selon la revendication 1, où la protéine pré-S de type sauvage est dérivée d'un VHB choisi dans le groupe constitué des sous-types adr, ayw, adw, adw2 et adyw.

3. Protéine pré-S du VHB selon la revendication 1, où la protéine pré-S est choisie dans le groupe constitué de pré-S-15m (SEQ ID NO : 9), pré-S-123m (SEQ ID NO : 10) et pré-S-dm (SEQ ID NO : 11).

4. Gène pré-S codant pour la protéine pré-S du VHB selon l'une quelconque des revendications 1 à 3.

5. Vecteur recombinant comprenant :
(a) un promoteur ;
(b) un gène pré-S selon la revendication 4 ; et
(c) un terminateur de la transcription.

6. Vecteur recombinant selon la revendication 5, dans lequel le vecteur est pIL20-pre-S (adr) tel que déposé auprès du KCTC sous le numéro d'accession KCTC 0987BP ou pIL20-pre-S (ayw) tel que déposé auprès du KCTC sous le numéro d'accession KCTC 1004BP.

7. Transformant comprenant le vecteur recombinant selon la revendication 5.

8. Transformant selon la revendication 7, où le transformant est une levure.

9. Transformant selon la revendication 7, où le transformant est choisi dans le groupe constitué de Saccharomyces cerevisiae 2805/pIL20-pre-S (KCTC 0987BP) et Saccharomyces cerevisiae 2805/pIL20-pre-S (ayw) (KCTC 1004BP).

10. Protéine pré-S recombinante produite à partir du transformant selon la revendication 7.

11. Adjuvant comprenant une protéine pré-S du VHB selon l'une quelconque des revendications 1 à 3.

12. Vaccin contre le VHB comprenant une protéine pré-S du VHB selon l'une quelconque des revendications 1 à 3.

13. Vaccin contre le VHB selon la revendication 12, où le vaccin contre le VHB comprend en outre un antigène S du VHB.

14. Composition diagnostique pour détecter des anticorps dirigés contre le VHB, les antigènes de surface du VHB ou les antigènes codés par le gène pré-S, où la composition diagnostique comprend l'une choisie dans le groupe constitué des protéines pré-S du VHB selon l'une quelconque des revendications 1 à 3.

15. Procédé de production d'une protéine pré-S du VHB, comprenant :
(a) l'insertion d'un gène pré-S selon la revendication 4 dans un vecteur ;
(b) la transfection du vecteur abritant le gène pré-S selon la revendication 4 dans une cellule hôte ; et
(c) la production de la protéine pré-S par la culture d'un transformant dans un milieu.

16. Utilisation d'une protéine pré-S du VHB selon l'une quelconque des revendications 1 à 3 pour préparer un adjuvant amplifiant un anticorps contre un antigène dérivé du VHB chez un mammifère ou un oiseau.

17. Utilisation selon la revendication 16, où le mammifère est choisi dans le groupe constitué d'un être humain, d'un animal de bétail, d'un animal de test de laboratoire, d'un animal domestique et d'un animal sauvage en captivité.

18. Utilisation selon la revendication 16, où l'oiseau est un poulet ou une autre volaille.

19. Utilisation d'une protéine pré-S du VHB selon l'une quelconque des revendications 1 à 3 pour préparer un vaccin pour générer une immunité pour le virus de l'hépatite B.

20. Utilisation selon la revendication 19, où le vaccin contre le VHB comprend en outre un antigène S du VHB.


### Sequence Listing

<110> Dobeel Corporation
<120> PRE S PROTEIN OF HEPATITIS B VIRUS (HBV) AS AN ADJUVANT AND A COMPONENT OF HBV VACCINE
<150> KR 10-2001-29002
   <151> 2001-05-25
<160> 11
<170> KopatentIn 1.71
<210> 1
   <211> 522
   <212> DNA
   <213> HBV(adr subtype) pre S gene
<400> 1
   atgggaggtt ggtcttccaa acctcgacaa ggcatgggga cgaatctttc tgttcccaat      60
   cctctgggat tctttcccga tcaccagttg gaccctgcgt tcggagccaa ctcaaacaat     120
   ccagattggg acttcaaccc caacaaggat cactggccag aggcaaatca ggtaggagtg     180
   ggagcattcg ggccagggtt caccccacca cacggcggtc ttttggggtg gagccctcag     240
   gctcagggca tattgacaac agtgccagca gcgcctcctc ctgcctccac caatcggcag     300
   tcaggaagac agcctactcc catctctcca cctctaagag acagtcatcc tcaggccatg     360
   cagtggaact ccaccacatt ccaccaagct ctgctagatc ccagagtgag gggcctatat      420
   tttcctgctg gtggctccag ttccggaaca gtaaaccctg ttccgactac tgcctcaccc     480
   atatcgtcaa tcttctcgag gactggggac cctgcaccga ac                        522
<210> 2
   <211> 522
   <212> DNA
   <213> HBV(ayw subtype) pre S gene
<400> 2
   atgggaggtt ggtcttccaa acctcgacaa ggcatggggc agaatctttc caccagcaat 60
   cctctgggat tctttcccga ccaccagttg gatccagcct tcagagcaaa caccgcaaat 120
   ccagattggg acttcaatcc caacaaggac acctggccag acgccaacaa ggtaggagct 180
   ggagcattcg ggctgggatt caccccacca cacggaggcc ttttggggtg gagccctcag 240
   gctcagggca tactagaaac gttgccagca aatccgcctc ctgcctctac caatcgccag 300
   tcaggaaggc agcctacccc gctgtctcca cctttgagaa acactcatcc tcaggccatg 360
   cagtggaact ccacaacctt ccaccaaact ctgcaagatc ccagagtgag aggcctgtat 420
   ttccctgctg gtggctccag ttcaggaaca gtaaaccctg ttccgactac tgtctctccc 480
   atatcgtcaa tcttctcgag gattggggac cctgcgctga ac 522
<210> 3
   <211> 522
   <212> DNA
   <213> HBV(adw subtype) pre S gene
<400> 3
   atgggaggtt ggtcatcaaa acctcgcaaa ggcatgggga cgaacctttc tgttcccaac 60
   cctctgggat tctttcccga tcatcagttg gaccctgcat tcggagccaa ttcaaacaat 120
   ccagattggg acttcaaccc catcaaggac cactggccac aagccaacca ggtaggagtg 180
   ggagcatttg ggccagggtt cactccccca cacggaggtg ttttggggtg gagccctcag 240
   gctcagggca tattggccac cgtgccagcg atgcctcctc ctgcctccac caatcggcag 300
   tcaggaaggc agcctactcc catctctcca cctctaagag acagtcatcc tcaggccatg 360
   cagtggaatt ccacagcttt ccaccaagct ctgcaagatc ccagagtcag gggcctgtat 420
   tttcctgctg gtggctccag ttcaggaaca ctcaaccctg ttccaactat tgcctctcac 480
   atctcgtcaa tctcctcgag gattggggac cctgcaccga ac 522
<210> 4
   <211> 174
   <212> PRT
   <213> HBV(adr subtype) pre S protein
<400> 4
   Met Gly Gly Trp Ser Ser Lys Pro Arg Gln Gly Met Gly Thr Asn Leu
   1               5                   10                  15
   Ser Val Pro Asn Pro Leu Gly Phe Phe Pro Asp His Gln Leu Asp Pro
               20                  25                  30
   Ala Phe Gly Ala Asn Ser Asn Asn Pro Asp Trp Asp Phe Asn Pro Asn
           35                  40                  45
   Lys Asp His Trp Pro Glu Ala Asn Gln Val Gly Val Gly Ala Phe Gly
       50                  55                  60
   Pro Gly Phe Thr Pro Pro His Gly Gly Leu Leu Gly Trp Ser Pro Gln
   65                  70                  75                  80
   Ala Gln Gly Ile Leu Thr Thr Val Pro Ala Ala Pro Pro Pro Ala Ser
                   85                  90                  95
   Thr Asn Arg Gln Ser Gly Arg Gln Pro Thr Pro Ile Ser Pro Pro Leu
               100                 105                 110
   Arg Asp Ser His Pro Gln Ala Met Gln Trp Asn Ser Thr Thr Phe His
           115                 120                 125
   Gln Ala Leu Leu Asp Pro Arg Val Arg Gly Leu Tyr Phe Pro Ala Gly
       130                 135                 140
   Gly Ser Ser Ser Gly Thr Val Asn Pro Val Pro Thr Thr Ala Ser Pro
   145                 150                 155                 160
   Ile Ser Ser Ile Phe Ser Arg Thr Gly Asp Pro Ala Pro Asn
                   165                 170
<210> 5
   <211> 174
   <212> PRT
   <213> HBV(ayw subtype) pre S protein
<400> 5
   Met Gly Gly Trp Ser Ser Lys Pro Arg Gln Gly Met Gly Gln Asn Leu
   1               5                   10                  15
   Ser Thr Ser Asn Pro Leu Gly Phe Phe Pro Asp His Gln Leu Asp Pro
               20                  25                  30
   Ala Phe Arg Ala Asn Thr Ala Asn Pro Asp Trp Asp Phe Asn Pro Asn
           35                  40                  45
   Lys Asp Thr Trp Pro Asp Ala Asn Lys Val Gly Ala Gly Ala Phe Gly
       50                  55                  60
   Leu Gly Phe Thr Pro Pro His Gly Gly Leu Leu Gly Trp Ser Pro Gln
   65                  70                  75                  80
   Ala Gln Gly Ile Leu Glu Thr Leu Pro Ala Asn Pro Pro Pro Ala Ser
                   85                  90                  95
   Thr Asn Arg Gln Ser Gly Arg Gln Pro Thr Pro Leu Ser Pro Pro Leu
               100                 105                 110
   Arg Asn Thr His Pro Gln Ala Met Gln Trp Asn Ser Thr Thr Phe His
           115                 120                 125
   Gln Thr Leu Gln Asp Pro Arg Val Arg Gly Leu Tyr Phe Pro Ala Gly
       130                 135                 140
   Gly Ser Ser Ser Gly Thr Val Asn Pro Val Pro Thr Thr Val Ser Pro
   145                 150                 155                 160
   Ile Ser Ser Ile Phe Ser Arg Ile Gly Asp Pro Ala Leu Asn
                   165                 170
<210> 6
   <211> 174
   <212> PRT
   <213> HBV(adw subtype) pre S protein
<400> 6
   Met Gly Gly Trp Ser Ser Lys Pro Arg Lys Gly Met Gly Thr Asn Leu
   1               5                   10                  15
   Ser Val Pro Asn Pro Leu Gly Phe Phe Pro Asp His Gln Leu Asp Pro
               20                  25                  30
   Ala Phe Gly Ala Asn Ser Asn Asn Pro Asp Trp Asp Phe Asn Pro Ile
           35                  40                  45
   Lys Asp His Trp Pro Gln Ala Asn Gln Val Gly Val Gly Ala Phe Gly
       50                  55                  60
   Pro Gly Phe Thr Pro Pro His Gly Gly Val Leu Gly Trp Ser Pro Gln
   65                  70                  75                  80
   Ala Gln Gly Ile Leu Ala Thr Val Pro Ala Met Pro Pro Pro Ala Ser
                   85                  90                  95
   Thr Asn Arg Gln Ser Gly Arg Gln Pro Thr Pro Ile Ser Pro Pro Leu
               100                 105                 110
   Arg Asp Ser His Pro Gln Ala Met Gln Trp Asn Ser Thr Ala Phe His
           115                 120                 125
   Gln Ala Leu Gln Asp Pro Arg Val Arg Gly Leu Tyr Phe Pro Ala Gly
       130                 135                 140
   Gly Ser Ser Ser Gly Thr Leu Asn Pro Val Pro Thr Ile Ala Ser His
   145                 150                 155                 160
   Ile Ser Ser Ile Ser Ser Arg Ile Gly Asp Pro Ala Pro Asn
                   165                 170
<210> 7
   <211> 39
   <212> DNA
   <213> primer
<400> 7
   gtctctagac aagagaatgg gaggttggtc ttccaaacc 39
<210> 8
   <211> 37
   <212> DNA
   <213> primer
<400> 8
   atcggatccc tagttcggtg cagggtcccc agtcctc 37
<210> 9
   <211> 174
   <212> PRT
   <213> PreS-15m protein
<400> 9
   Met Gly Gly Trp Ser Ser Lys Pro Arg Gln Gly Met Gly Thr His Leu
   1 5 10 15
   Ser Val Pro Asn Pro Leu Gly Phe Phe Pro Asp His Gln Leu Asp Pro
   20 25 30
   Ala Phe Gly Ala Asn Ser Asn Asn Pro Asp Trp Asp Phe Asn Pro Asn
   35 40 45
   Lys Asp His Trp Pro Glu Ala Asn Gln Val Gly Ala Gly Ala Phe Gly
   50 55 60
   Pro Gly Phe Thr Pro Pro His Gly Gly Leu Leu Gly Trp Ser Pro Gln
   65 70 75 80
   Ala Gln Gly Ile Leu Thr Thr Val Pro Ala Ala Pro Pro Pro Ala Ser
   85 90 95
   Thr Asn Arg Gln Ser Gly Arg Gln Pro Thr Pro Ile Ser Pro Pro Leu
   100 105 110
   Arg Asp Ser His Pro Gln Ala Met Gln Trp Asn Ser Thr Thr Phe His
   115 120 125
   Gln Ala Leu Leu Asp Pro Arg Val Arg Gly Leu Tyr Phe Pro Ala Gly
   130 135 140
   Gly Ser Ser Ser Gly Thr Val Asn Pro Val Pro Thr Thr Ala Ser Pro
   145 150 155 160
   Ile Ser Ser Ile Phe Ser Arg Thr Gly Asp Pro Ala Pro Asn
   165 170
<210> 10
   <211> 174
   <212> PRT
   <213> PreS-123m protein
<400> 10
   Met Gly Gly Trp Ser Ser Lys Pro Arg Gln Gly Met Gly Thr Asn Leu
    1               5                   10                  15
   Ser Val Pro Asn Pro Leu Gly Phe Phe Pro Asp His Gln Leu Asp Pro
               20                  25                  30
   Ala Phe Gly Ala Asn Ser Asn Asn Pro Asp Trp Asp Phe Asn Pro Asn
           35                  40                  45
   Lys Asp His Trp Pro Glu Ala Asn Gln Val Gly Ala Gly Ala Phe Gly
       50                  55                  60
   Pro Gly Phe Thr Pro Pro His Gly Gly Leu Leu Gly Trp Ser Pro Gln
   65                  70                  75                  80
   Ala Gln Gly Ile Leu Thr Thr Val Pro Ala Ala Pro Pro Pro Ala Ser
                   85                  90                  95
   Thr Asn Arg Gln Ser Gly Arg Gln Pro Thr Pro Ile Ser Pro Pro Leu
               100                 105                 110
   Arg Asp Ser His Pro Gln Ala Met Gln Trp His Ser Thr Thr Phe His
           115                 120                 125
   Gln Ala Leu Leu Asp Pro Arg Val Arg Gly Leu Tyr Phe Pro Ala Gly
       130                 135                 140
   Gly Ser Ser Ser Gly Thr Val Asn Pro Val Pro Thr Thr Ala Ser Pro
   145                 150                 155                 160
   Ile Ser Ser Ile Phe Ser Arg Thr Gly Asp Pro Ala Pro Asn
                   165                 170
<210> 11
   <211> 174
   <212> PRT
   <213> PreS-dm protein
<400> 11
   Met Gly Gly Trp Ser Ser Lys Pro Arg Gln Gly Met Gly Thr His Leu
    1               5                  10                  15
   Ser Val Pro Asn Pro Leu Gly Phe Phe Pro Asp His Gln Leu Asp Pro
               20                  25                  30
   Ala Phe Gly Ala Asn Ser Asn Asn Pro Asp Trp Asp Phe Asn Pro Asn
           35                  40                  45
   Lys Asp His Trp Pro Glu Ala Asn Gln Val Gly Ala Gly Ala Phe Gly
       50                  55                  60
   Pro Gly Phe Thr Pro Pro His Gly Gly Leu Leu Gly Trp Ser Pro Gln
   65                  70                  75                  80
   Ala Gln Gly Ile Leu Thr Thr Val Pro Ala Ala Pro Pro Pro Ala Ser
                   85                  90                  95
   Thr Asn Arg Gln Ser Gly Arg Gln Pro Thr Pro Ile Ser Pro Pro Leu
               100                 105                 110
   Arg Asp Ser His Pro Gln Ala Met Gln Trp His Ser Thr Thr Phe His
           115                 120                 125
   Gln Ala Leu Leu Asp Pro Arg Val Arg Gly Leu Tyr Phe Pro Ala Gly
       130                 135                 140
   Gly Ser Ser Ser Gly Thr Val Asn Pro Val Pro Thr Thr Ala Ser Pro
   145                 150                 155                 160
   Ile Ser Ser Ile Phe Ser Arg Thr Gly Asp Pro Ala Pro Asn
                   165                 170